Europäisches Patentamt

European Patent Office

Office européen des brevets

(11) Publication number: **0 070 687**
**B1**

(12) **EUROPEAN PATENT SPECIFICATION**

(45) Date of publication of patent specification: **16.10.85**

(51) Int. Cl.⁴: **G 01 N 33/58, G 01 N 21/76**

(21) Application number: **82303701.5**

(22) Date of filing: **14.07.82**

(54) **Light-emitting polynucleotide hybridization diagnostic method.**

(30) Priority: **17.07.81 US 284399**
**17.07.81 US 284401**

(43) Date of publication of application:
**26.01.83 Bulletin 83/04**

(45) Publication of the grant of the patent:
**16.10.85 Bulletin 85/42**

(84) Designated Contracting States:
**BE CH DE FR GB IT LI NL**

(56) References cited:
**WO-A-81/01883**
**GB-A-2 019 408**
**US-A-4 230 797**
**US-A-4 261 893**
**US-A-4 302 204**

(73) Proprietor: **AMOCO CORPORATION**
**200 East Randolph Drive**
**Chicago Illinois 60601 (US)**

(72) Inventor: **Heller, Michael James**
**30 W 057-104 Granada**
**Naperville Illinois 60540 (US)**
Inventor: **Morrison, Larry Edward**
**4913 Spencer**
**Lisle Illinois 60532 (US)**
Inventor: **Prevatt, William Dudley**
**1205 Grant Street**
**Downers Grove Illinois 60515 (US)**
Inventor: **Akin, Cavit**
**1462 Inverrary Drive**
**Naperville Illinois 60540 (US)**

(74) Representative: **Ritter, Stephen David et al**
**Mathys & Squire 10 Fleet Street**
**London EC4Y 1AY (GB)**

Courier Press, Leamington Spa, England.

## Description

### Background of the invention

Presently, nucleic acid hybridization assays are primarily used as a research tool for the detection and identification of a unique deoxyribonucleic acid (DNA) sequence or a specific gene in a complete DNA, a mixture of DNA's, or a mixture of DNA fragments. A number of variations of the technique exist as used in recombinant DNA research (*Methods in Enzymology*, Vol. 68, R. Wu (Ed.) pp. 379—469, 1979; and Dunn, A. R., and Sambrook, J., *Methods in Enzymology*, Vol. 65, Part 1, 468—478, 1980). One of the most widely used procedures is called the Southern blot filter hybridization method (Southern, E., J. Mol. Biol. *98*, 503, 1975). This procedure is usually used to identify a particular DNA fragment separated from a mixture of DNA fragments by electrophoresis. The procedure is generally carried out by subjecting a sample of DNA isolated from some organism to restriction endonuclease digestion and then electrophoresing the sample on a gel (agarose, acrylamide). The gel containing the separated DNA fragments is placed in contact (blotted) with a nitrocellulose filter sheet (or diazotized paper). The DNA fragments are transferred and become bound to the nitrocellulose sheet. The gel-transfer nitrocellulose sheet containing the DNA fragments is then heated (95°C) to denature the DNA. At ʼthis point the sheet is treated with a solution containing a denatured radio labeled ($32_p$) "specific DNA probe" and hybridization is allowed to take place. Hybridization can take from 3—48 hours, depending on given conditions, and unhybridized "specific DNA probe" is washed away. The nitrocellulose sheet is then placed on a sheet of X-ray film and allowed to expose, usually for several days at −70°C. The X-ray film is then developed. The exposed areas on the film identify which DNA fragments have hybridized and thus have a sequence similar to that of the "specific DNA probe".

This procedure, as well as most other variations, requires the use of radioisotopes and is obviously very complex and time consuming. Because of these and other problems, DNA hybridization assays have remained only as a tool for basic research and have not been generally used in applied or commercial areas, such as for clinical diagnostics.

It would be very desirable to have a hybridization method which would avoid some of these problems, particularly the use of radioisotopes. Hybridization assays could be a valuable diagnostic method for identification of pathogenic organisms and for the determination of antibiotic resistant traits (genes) in microorganisms. They could also be used for genetically based human cell typing systems and for diagnosis of genetic diseases.

### Summary of the invention

In general, the invention relates to a hybridization assay which is based on the inherent high fidelity of the base recognition process in complementary double-stranded (ds) nucleic acids (DNA, RNA, DNA-RNA, and synthetic polynucleotides). It involves a hybridization system that does not use radioisotopes, but instead uses chemiluminescent, fluorescent, or phosphorescent moieties which under proper conditions can provide sensitivity equal to that of radioisotopes but without the inherent problems. This invention has advantages over present methods which are too complex and time consuming to be used practically in clinical diagnostics.

In one aspect, the invention resides in a diagnostic method for determining the presence of and the identification of viruses, bacteria, and other microorganisms, as well as the existence of certain genetic traits by assaying for a target nucleotide sequence which is uniquely characteristic of the target microorganism or genetic trait being assayed.

In particular, the present invention provides a method for assaying a target single-stranded polynucleotide sequence in a single-stranded polynucleotide sample comprising (a) contacting an immobilized single-stranded polynucleotide sample on a support, under hybridization conditions, with light-labeled single-stranded polynucleotide reagent having a nucleotide sequence which is complementary to a representative portion of the target single-stranded polynucleotide sequence; (b) separating unhybridized single-stranded polynucleotide reagent from the immobilized sample; (c) exposing the immobilized sample to means for exciting the light label; and (d) detecting the light response from the sample.

The method of this invention is directed towards assaying single-stranded polynucleotides because of their ability to combine with complementary strands in a highly specific manner. The single-stranded polynucleotides useful for purposes of this invention are readily obtained from double-stranded polynucleotides by denaturing, i.e. splitting the double-stranded polynucleotide into two complementary strands. This is typically accomplished by heating to about 70—100°C for about 5—10 minutes. The "target" single-stranded polynucleotide sequence mentioned above is either of the two single-stranded polynucleotides obtained by denaturing that double-stranded polynucleotide (the target double-stranded polynucleotide) at which the diagnosis is ultimately directed, such as a particular gene. Similarly, the "single-stranded polynucleotide sample" can be obtained by isolating the double-stranded polynucleotides from the physiological sample to be diagnosed (blood, urine, other body fluids) and denaturing the double-stranded polynucleotides to form the corresponding single strands.

Immobilization of the sample single-stranded polynucleotides can be accomplished by any suitable method which does not inactive a significant number of the bases in the polynucleotide sequence, since a representative intact sequence must be available for base pairing with the reagent strands. The single-stranded polynucleotide sample can be attached or immobilized to a variety of supports, some of which include activated glass beads, polyacrylamide, agarose or Sephadex® beads, and cellulose. Numerous methods exist for coupling either terminal end of a polynucleotide to a given support (Weissback, A., and Poonian, M., *Methods in Enzymology*, Vol. XXXIV, Part B, 463—475, 1974). Also, derivatized forms of polynucleotides, for example, one carrying a terminal aminohexyl nucleotide, can be easily attached on a variety of supports (Mosbach, K., et al., *Methods of Enzymology*, Vol. XLIV, 859—886, 1976). Oligoribonucleotides may be immobilized on boronate derivates of various supports (Schott, H., et al., *Biochemistry*, 12, 932, 1973).

Hybridization conditions necessary to accomplish base pairing of the target single-stranded polynucleotide sequence in the sample (if present) and the single-stranded polynucleotide reagent are determined by a variety of factors, such as the nature of the light label attached to the reagent polynucleotide sequences, the size of the reagent polynucleotide sequences, the [G]+[C] (guanine plus cytosine) content of the reagent and sample polynucleotide sequences, and how the sample polynucleotide sequence is prepared. The light label can affect the temperature and salt concentration for the hybridization reaction. Chemiluminescent catalysts can be sensitive to temperatures and salt concentrations that absorber/emitter moieties can tolerate. Size of the reagent polynucleotide sequences affects the temperature and time for the hybridization reaction. Assuming similar salt and reagent concentrations, hybridization involving reagent polynucleotide sequences in the range of 10,000 to 100,000 nucleotides might require from 40 to 80 minutes to occur at 67°C, while hybridization involving 20 to 100 nucleotides would require from 50 to 30 minutes at 25°C. Similarly, the [G]+[C] content of the reagent and sample polynucleotide sequences affects the temperature and time for the hybridization reaction. Polynucleotide sequences with a high [G]+[C] content will hybridize at lower temperatures in a shorter period of time than polynucleotide sequences with a low [G]+[C] content. Finally, conditions used to prepare the sample polynucleotide sequence and maintain it in the single-stranded form can affect the temperature, time, and salt concentration of the hybridization reaction. The conditions for preparing the sample polynucleotide sequence are affected by polynucleotide length required and the [G]+[C] content. In general, the longer the sequence or the higher the [G]+[C] content, the higher the temperature and/or salt concentration required.

The single-stranded polynucleotide reagent must have a nucleotide sequence which is complementary to a representative portion of the target single-stranded polynucleotide sequence so as not to give a false indication of a positive test in the absence of the target single-stranded polynucleotide sequence in the sample. The term "representative" is intended to be an imprecise term since the desired accuracy of the method will vary with the purpose. Ideally, the reagent single-stranded polynucleotide will only pair with target single-stranded polynucleotide and will do so whenever the latter is present in the sample. If the target single-stranded polynucleotide base sequence is very unique, very high accuracy can be obtained. Preferably, the single-stranded polynucleotide reagents are prepared from the target double-stranded polynucleotide, which is denatured and separated into the two complementary single strands. Either single strand can be chosen as the reagent material to be light-labeled, but only one of them can actually be used in order to prevent the reagent from hybridizing with itself. The sample single-stranded polynucleotides need not be separated in this manner, however, in which case only half of the single-stranded polynucleotides present in the sample, which were derived from the target double-stranded polynucleotide, would be suitable hybridization candidates. If sequences are known, then reagent single-stranded polynucleotides can be made by organic synthetic techniques.

Light labels can be chemiluminescent, bioluminescent, phosphorescent, or fluorescent and can be attached to any point on the reagent single-stranded polynucleotide; however, terminal positions are more desirable. Suitable light labels which are molecular species participating in a chemiluminescent reaction include, without limitation, peroxidase and iron porphyrin derivatives. Suitable light labels which are molecular species particpating in a bioluminescent reaction include, without limitation, bacterial luciferase (either one or both luciferase and associated dehydrogenase), firefly luciferase, flavin mononucleotide (FMN), adenosine triphosphate (ATP), reduced nicotinamide adenine dinucleotide (NADH), reduced nicotinamide adenine dinucleotide phosphate (NADPH), and various long chain aldehydes (decyl aldehyde). Suitable fluorescent light labels include, without limitation, a variety of fluorescent nucleotides (ethenoadenosine nucleotides, etheno-cytidine nucleotides, or functionalized nucleotides (aminohexane adenosine nucleotides, mercurinucleotides) which could first be fluorescently labeled and then covalently attached to the reagent single-stranded polynucleotide. A variety of methods exist for preparing fluorescent derivatives of oligoribonucleotides (Annual Review Biophys. Bioeng., A. Faure and G. Thomas, pp. 175—195, 1981). Suitable phosphorescent light labels include, without limitation, 2-diketones such as 2,3-butadione 1-[carboxyphenyl]-1,2-pentanedione, and 1-phenyl-1,2-propanedione. Means for attaching the light labels to the reagent single-stranded polynucleotide segments are documented in the art.

In order to determine if hybridization has occurred and hence the presence of the target polynucleotide sequence in the sample, it is first necessary to remove the unhybridized light-labeled reagent. This is most simply accomplished by washing or flushing with appropriate buffer solution. Complete removal of unhybridized reagent is a goal to be attained, but is not likely since there will be some residual reagent which sticks to the immobilization support or possible partially hybridizes with the sample. These occurrences will cause "background" when measuring the light response and must be accounted for when analyzing the results of the test.

After the separating step, the immobilized sample must be exposed to a means for exciting the light labels, if present, which may have become immobilized through hybridization between the reagent and immobilized sample single-stranded polynucleotides. In the case of fluorescent or phosphorescent labels, the sample need only be irradiated with the appropriate light wavelength. This technique is well known in the art. In the case of the light labels which are molecular species participating in chemiluminescent or bioluminescent reactions, the sample must be exposed to a light reagent system which depends specifically on the light label which has been used. Such light reagents are also well known in the art. For example, the following reactions illustrate how a light response is elicited using three different catalysts as the light labels, namely bacterial luciferase, firefly luciferase, and peroxidase:

$$NADH_2+FMN+RCHO+O_2 \xrightarrow{\text{bacterial luciferase}}$$

$$NAD+FMN+RCOOH+H_2O+hv$$

$$luciferin+ATP+O_2 \xrightarrow[Mg++]{\text{firefly luciferase}}$$

$$oxyluciferin+AMP+CO_2+PPi+hv$$

$$H_2O_2+luminol \xrightarrow{\text{peroxidase}} oxyluminol+H_2O+N_2+hv$$

In the presence of an excess of reactants, the light response will vary with the number of light-labeled reagent single-stranded polynucleotide segments which have become hybridized with the immobilized sample. The choice of the label will be determined by ease of labeling and the economics of the light reagent system.

The detection of the light response can be accomplished with numerous detection devices which are commercially available. For example, using photomultiplier detection devices, $NADH_2$ levels of $10^{-3}$—$10^{-9}$ mg/ml, ATP levels of $10^{-3}$— $10^{-11}$ mg/ml, and $H_2O_2$ levels of $10^{-3}$—$10^{-7}$ mg/ml can be measured.

As mentioned above, it is necessary to immobilize the sample single-stranded polynucleotide on a support. In addition to the aforementioned methods of immobilization, the single-stranded polynucleotide sample may be immobilized by contacting the sample under hybridization conditions with an immobilized single-stranded polynucleotide said first single-stranded polynucleotide including a sequence which is complementary to a first portion of the target single-stranded polynucleotide. When target single-stranded polynucleotide is present in the sample it hybridizes with the immobilized single-stranded polynucleotide reagent. The target single-stranded polynucleotide then serves as a template for hybridization with the light-labeled second single-stranded polynucleotide reagent which includes a polynucleotide sequence which is complementary to a substantially mutually exclusive portion of the target single-stranded polynucleotide relative to said first single-stranded polynucleotide. This system has the advantage of improved specificity because it involves two hybridization processes.

The first and second single-stranded polynucleotide reagent sequences must consist essentially of bases which are complementary to the base sequence of the target single-stranded polynucleotide. It is necessary that the first and second sequences be complementary to substantially mutually exclusive portions of the target single-stranded polynucleotide. In other words, upon hybridization with the target polynucleotide the first and second reagent sequences should not compete for the same base sequence to the extent that hybridization is prevented. In some cases, the first and second segments will line up head to tail (3' end to 5' end) with no overlap and with few or no basepairing spaces left between them. However, in other cases it will be highly desirable (because it would improve specificity) that the first and second single-stranded polynucleotide reagent sequences be complementary to two widely separated but unique sequences on the target single-stranded polynucleotide.

First and second single-stranded polynucleotide reagent sequences can be prepared from appropriate restriction endonuclease treatment of target nucleic acids from the organism of interest or, in cases where the base sequence of a unique portion is known, they can also be synthesized by organic synthetic techniques (Stawinski, J., et al., Nucl. Acids Res. 4, 353, 1977; Gough, G. R. et al., Nucl. Acids Res. 6, 1557, 1979; Gough, G. R. et al., Nucl. Acids Res. 7, 1955, 1979; Narang, S. A., *Methods in Enzymology*, Vol. 65, Part I, 610—620, 1980).

The size of the reagent sequences can be from 10 nucleotides to 100,000 nucleotides in length. Hybridized systems having fewer than 10 nucleotides are not stable and will begin to denature about 20°C. Above 100,000 nucleotides, one finds that hybridization (renaturation) becomes a much slower and incomplete process, (See *Molecular Genetics*, G. S. Stent and R. Calender, pp. 213—219, 1971). Ideally the reagent sequences should be from 20 to 10,000 nucleotides. Smaller nucleotide sequences (20—100) would lend themsleves to production by automated organic synthetic techniques. Sequences from 100—100,000 nucleotides could be obtained from appropriate restriction endonuclease treatments. Ideally small sequences should be fluorescent labeled, while larger sequences could be chemiluminescent labeled. The labeling of the smaller sequences with the relatively bulky chemiluminescent moieties may in some cases interfere with the hybridization process.

### Claims

1. A method for assaying a target single-stranded polynucleotide sequence in a single-stranded polynucleotide sample comprising:

a) contacting an immobilized single-stranded polynucleotide sample on a support, under hybridization conditions, with light-labeled single-stranded polynucleotide reagent having a nucleotide sequence which is complementary to a representative portion of the target single-stranded polynucleotide sequence;

b) separating unhybridized single-stranded polynucleotide reagent from the immobilized sample;

c) exposing the immobilized sample to means for exciting the light label; and

d) detecting the light response from the sample.

2. A method according to Claim 1 wherein (a) the single-stranded polynucleotide sample is immobilized by contacting the sample, under hybridization conditions, with immobilized, single-stranded polynucleotide including a sequence which is complementary to a first portion of the target single-stranded polynucleotide sequence, and (b) the light-labeled single-stranded polynucleotide reagent includes a polynucleotide sequence which is complementary to a second portion of the target single-stranded polynucleotide sequence, said first and second portions being mutually exclusive.

3. A method according to Claim 1 or Claim 2 wherein the light label is a molecular species participating in a chemiluminescent reaction.

4. A method according to Claim 3 wherein the light label is selected from peroxidase and iron porphyrin derivatives.

5. A method according to Claim 1 or Claim 2 wherein the light label is a fluorescent label.

6. A method according to Claim 5 wherein the fluorescent label is selected from ethenoadenosine nucleotides, ethenocytidine nucleotides, amino-hexane adenosine nucleotides, and mercurinucleotides.

7. A method according to Claim 1 or Claim 2 wherein the light label is a molecular species participating in a bioluminescent reaction.

8. A method according to Claim 7 wherein the light label is selected from bacterial luciferase, firefly luciferase, flavin mononucleotide, adenosine triphosphate, reduced nicotinamide adenine dinucleotide, reduced nicotinamide adenine dinucleotide phosphate, and decyl aldehyde.

9. A method according to Claim 1 or Claim 2 wherein the light label is a phosphorescent label.

10. A method according to Claim 9 wherein the phosphorescent label is selected from 2,3-butadione, 1-[carboxyphenyl]-1,2-pentanedione, and 1-phenyl-1,2 propanedione.

### Patentansprüche

1. Verfahren zur Prüfung einer das gewünschte Ziel bildenden einsträngigen Polynucleotidsequenz in einer einsträngigen Polynucleotidprobe, gekennzeichnet, durch

a) Behandlung einer auf einem Träger befindlichen, immobilisierten einsträngigen Polynucleotidprobe unter Hybridisierungsbedingungen mit einem mit einer Lichtmarkierung versehenen einsträngigen Polynucleotidreagenz, das eine Nucleotidsequenz hat, die zu einem repräsentativen Teil der als Ziehl gewünschten einsträngigen Polynucleotidsequenz komplementär ist,

b) Abtrennung des nichthybridisierten einsträngigen Polynucleotidreagenz von der immobilisierten Probe,

c) Behandlung der immobilisierten Probe mit einem Mittel zur Anregung der Lichtmarkierung und

d) Detektion des Lichtsignals der Probe.

2. Verfahren nach Anspruch 1, dadurch gekennzeichnet, daß

a) die einsträgnige Polynucleotidprobe durch Behandlung der Probe unter Hybridisierungsbedingungen mit einem immobilisierten einsträngigen Polynucleotid immobilisiert ist, das eine Sequenz enthält, die zu einem ersten Teil der das gewünschte Ziel bildenden einsträngigen Polynucleotidsequenz komplementär ist, und

b) das mit einer Lichtmarkierung versehene einsträngige Polynucleotidreagenz eine Polynucleotides—quenz enthält, die zu einem zweiten Teil der das gewünschte Ziel bildenden einsträngigen Polynucleotidsequenz komplementär ist, wobei sich das erste Teil und das zweite Teil gegenseitig ausschließen.

3. Verfahren nach Anspruch 1 oder 2, dadurch gekennzeichnet, daß die Lichtmarkierung aus einer Molekülart besteht, die an einer Chemilumineszenzreaktion teilnimmt.

4. Verfahren nach Anspruch 3, dadurch gekennzeichnet, daß die Lichtmarkierung ausgewählt ist aus Peroxidasederivaten und Eisenporphyrinderivaten.

5. Verfahren nach Anspruch 1 oder 2, dadurch gekennzeichnet, daß die Lichtmarkierung eine Fluoreszenzmarkierung ist.

6. Verfahren nach Anspruch 5, dadurch gekennzeichnet, daß die Fluoreszenzmarkierung ausgewählt ist aus Ethenoadenosinnucleotiden,

Ethenocytidinnucleotiden, Aminohexanadenosinnucleotiden und Mercurinucleotiden.

7. Verfahren nach Anspruch 1 oder 2, dadurch gekennzeichnet, daß die Lichtmarkierung eine Molekülart ist, die an einer Biolumineszenzreaktion teilnimmt.

8. Verfahren nach Anspruch 7, dadurch gekennzeichnet, daß die Lichtmarkierung ausgewählt ist aus bakterieller Luciferase, Leuchtkäferluciferase, Flavinmononucleotid, Adenosintriphosphat, reduziertem Nicotinamidadenindinucleotid, reduziertem Nicotinamidadenindinucleotidphosphat und Decylaldehyd.

9. Verfahren nach Anspruch 1 oder 2, dadurch gekennzeichnet, daß die Lichtmarkierung eine Phosphoreszenzmarkierung ist.

10. Verfahren nach Anspruch 9, dadurch gekennzeichnet, daß die Phosphoreszenzmarkierung ausgewählt ist aus 2,3-Butadion, 1-[Carboxyphenyl]-1,2-pentandion und 1-Phenyl-1,2-propandion.

## Revendications

1. Un procédé pour la détermination d'une séquence de polynucléotide à brin unique cible dans un échantillon de polynucléotides à brin unique comprenant:

a) le contact d'un échantillon de polynucléotides à brin unique immobilisé sur un support, dans des conditions d'hybridation, avec un polynucléotide à brin unique réactif photomarqué ayant une séquence de nucléotides qui est complèmentaire d'une portion représentative de la séquence de polynucléotide à brin unique cible;

b) la séparation du polynucléotide à brin unique réactif non hybridé d'avec l'échantillon immobilisé;

c) l'exposition de l'échantillon immobilisé à un moyen d'excitation du photomarquer; et

d) la détection de la réponse lumineuse de l'échantillon.

2. Un procédé selon la revendication 1, dans lequel (a) l'échantillon de polynucléotides à brin unique est immobilisé par contact de l'échantillon, dans des conditions d'hybridation, avec un polynucléotide à brin unique immobilisé comprenant une séquence qui est complémentaire d'une première portion de la séquence de polynucléotide à brin unique cible et (b) le polynucléotide à brin unique réactif photomarqué comprend une séquence de polynucléotide qui est complémentaire d'une seconde portion de la séquence de polynucléotide à brin unique cible, ladite première et ladite seconde portions s'excluant mutuellement.

3. Un procédé selon la revendication 1 ou 2, dans lequel le photomarqueur est une espèce moléculaire participant à une réaction chimio-luminescente.

4. Un procédé selon la revendication 3, dans lequel le photomarqueur est choisi parmi une peroxydase et les dérivés de porphyrine contenant du fer.

5. Un procédé selon la revendication 1 ou la revendication 2, dans lequel le photomarqueur est un marqueur fluorescent.

6. Un procédé selon la revendication 5, dans lequel le marqueur fluorescent est choisi parmi les éthénoadénosine-nucléotides, les éthéno-cytidine-nucléotides, les amino-hexane-adénosine-nucléotides et les mercurinucléotides.

7. Un procédé selon la revendication 1 ou la revendication 2, dans lequel le photomarqueur est une espèce moléculaire participant à une réaction bioluminescente.

8. Un procédé selon la revendication 7, dans lequel le photomarqueur est choisi parmi une luciférase bactérienne, la luciférase de luciole, le flavine-mononucléotide, l'adénosine-triphosphate, le nicotinamide-adénine-dinucléotide réduit, le nicotinamide-adénine-dinucléotide-phosphate réduit et le décyladéhyde.

9. Un procédé selon la revendication 1 ou la revendication 2, dans lequel le photomarqueur est un marqueur phosphorescent.

10. Un procédé selon la revendication 9, dans lequel le marqueur phosphorescent est choisi parmi la 2,3-butanedione, la 1-{carboxy-phényl}-1,2-pentanedione et la 1-phényl-1,2-propanedione.